(19) Europäisches Patentamt
European Patent Office
Office européen des brevets

(11) Numéro de publication: **0 171 310**
**B1**

(12) **FASCICULE DE BREVET EUROPEEN**

(45) Date de publication du fascicule du brevet: **04.04.90**

(51) Int. Cl.$^5$: **A 01 H 1/02**

(21) Numéro de dépôt: **85401350.5**

(22) Date de dépôt: **03.07.85**

(54) **Procédé de préparation d'haploides doubles de céréales.**

(30) Priorité: **04.07.84 FR 8410631**

(43) Date de publication de la demande:
**12.02.86 Bulletin 86/07**

(45) Mention de la délivrance du brevet:
**04.04.90 Bulletin 90/14**

(84) Etats contractants désignés:
**AT BE CH DE FR GB IT LI LU NL SE**

(56) Documents cités:
**EP-A-0 127 313**

(73) Titulaire: **Etablissement Public dit: CENTRE NATIONAL DE LA RECHERCHE SCIENTIFIQUE (CNRS)**
**15, Quai Anatole France**
**F-75007 Paris (FR)**

(73) Titulaire: **UNIVERSITE PARIS-SUD (PARIS XI)**
**15, rue Georges Clémenceau**
**F-91405 Orsay Cédex (FR)**

(73) Titulaire: **INSTITUT NATIONAL DE LA RECHERCHE AGRONOMIQUE (INRA)**
**149, rue de Grenelle**
**F-75341 Paris Cedex 07 (FR)**

(72) Inventeur: **Picard, Emmanuel**
**8, Allée des Bathes**
**F-91940 Les Ulis (FR)**

(74) Mandataire: **Warcoin, Jacques et al**
**Cabinet Régimbeau 26, avenue Kléber**
**F-75116 Paris (FR)**

**EP 0 171 310 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne un procédé pour la production de lignées haploïdes doublées de céréales.

Une plante haploïde doublée par définition est une plante qui provient d'une déviation du processus normal de reproduction sexuée. Dans les plantes à fleurs, le cycle végétatif est appelé 2n (diploïde), chaque cellule de l'organisme se divise par mitose. La mitose est un processus par lequel une cellule se divise pour donner 2 cellules filles qui portent chacune la même information génétique que la cellule mère et également le même nombre de chromosomes, unité d'information héréditaire.

Pour initier le cycle sexuel, il est nécessaire d'avoir un processus de réduction du nombre des chromosomes (la méïose) pour donner naissance à des cellules gamétiques portant n chromosomes (nombre haploïde).

Dans les plantes à fleurs, la reproduction sexuée implique une double fécondation. Une cellule mâle s'unit avec une ovule pour former un zygote qui produit l'embryon, en restaurant le nombre de chromosomes somatiques. Une autre cellule mâle s'unit avec les 2 noyaux polaires, en général fusionnés en un noyau central. La cellule résultante triploïde conduit à l'endosperme. Dans certaine cas, cette double fécondation ne se produit pas et l'une des deux cellules gamétiques, se divise seule pour donner lieu à une plante haploïde. Ces plantes haploïdes sont très intéressantes dans les domaines de la génétique et de l'amélioration des plantes car, après le doublement chromosomique, les informations héréditaires seront identiques sur les deux chromosomes de chaque paire. De cette façon, l'information génétique sera fixée et les haploïdes doublés seront des outils permettant de simplifier et d'accélérer les processus de sélection.

Dans le cadre de la présente description, la terminologie "haploïde doublé" désignera une lignée diploïde de blé, par exemple descendant de la phase haploïde de cette espèce (gamètes mâles ou femelles).

Les recherches sur l'haplodiploïdisation des plantes supérieures ont été marquées au cours des 15 dernières années par plusieurs découvertes qui ont ouvert des étapes importantes dans ce domaine. Les travaux de Guha et Maheshwari (1964) sur la culture d'anthères *in vitro* ont incontestablement provoqué un très grand nombre de recherches sur cette technique d'obtention d'haploïdes à partir des gamètes mâles (androgénèse *in vitro*). A la même époque les croisements interspécifiques et intergénériques, ainsi que des systèmes génétiques inducteurs, étaient déjà reconnus comme inducteurs d'haploïdes, soit qu'ils provoquaient une parthénogénèse, une semigamie ou une élimination chromosomique (Hougas et Peloquin, 1957; Turcotte et Feaster, 1963; Kasha et Kao, 1970; Chase, 1949).

Le cas de l'orge est, à ce titre, tout à fait remarquable puisque le croisement interspécifique de cette espèce avec *Hordeum bulbosum* a ouvert une voie très efficace d'obtention et d'utilisation des haploïdes doublés en sélection (Kasha et Reinbergs, 1980).

Puis les travaux de San Noeum (1976) sur l'orge ouvraient une autre étape, celle de la gynogénèse *in vitro*.

Ainsi, ce sont les techniques de culture *in vitro* appliquées aux gamétophytes, cette "génération oubliée" (Heslop-Harrison, 1980) qui ont permis d'étudier au niveau haploïde et à l'état homozygote qui en découle, le plus grand nombre d'espèces. Elles continuent de marquer profondément les recherches en génétique végétale, non seulement en ce qui concerne les possibilités remarquables qu'elles ouvrent pour la sélection, mais également parce qu'elles sont source d'une nouvelle variabilité génétique (Larkin et Scowcroft, 1981).

Cependant, si on analyse les résultats concrets obtenus par l'utilisation de l'haplodiploïdisation en amélioration des plantes, on doit reconnaître encore ajuourd'hui que cette méthode n'a donné de résultats pratiques que chez très peu d'espèces. On en trouve en effet chez le maïs, la pomme de terre, l'orge et le cotonnier, pour les techniques n'utilisant pas l'androgénèse in vitro.

Quant aux haploïdes doublés androgénétiques sur lesquels les essais agronomiques sont déjà très avancés ou qui sont déjà inscrits comme cultivars, on peut en citer quelques uns chez l'asperge, la pomme de terre, le colza, le riz, le blé, l'orge, l'hévéa et le tabac (Loo Chiwei, 1982).

Les causes de cette lenteur du transfert de l'haplométhode au niveau de l'application sont à rechercher, en partie, dans les limites actuelles des techniques de culture *in vitro* qui sont: a) la grande spécificité génotypique de réponse à la culture *in vitro*; b) les faibles pourcentages de microspores ou de cellules du sac embryonnaire se dédifférenciant pour donner naissance à des embryons, et c) la perte importante de ces embryons quand on veut les conduire vers une organogénèse complète.

Le blé tendre fait donc partie de ces espèces où l'effort déployé est très important, étant donné l'enjeu, pour surmonter ces problèmes. Dans ce contexte les premiers résultats obtenus dans le cadre de la présente invention ouvrent une nouvelle voie d'obtention d'haploïdes doublés *in vivo* chez le blé, grâce à l'utilisation d'un agent chimique d'hybridation (A.C.H.).

Ce nouveau procédé se caractérise, en effet, par une haploïde induite *in vivo* à une fréquence suffisamment élevée pour en envisager l'application, cas qui jusqu'à présent ne s'était pas présenté (Lacadena, 1974).

Un procéde d'obtention d'haploïdes doublés de céréales est décrit dans EP—A—0 127 313 dans lequel les plantes sont traitées avec un agent chimique d'haploïdisation entre le stage 6 à 10.5 selon l'échelle de Feekes.

La présente invention concerne un procédé d'obtention d'haploïdes doublés de céréales dans lequel:

a) on traite les plantes par un agent chimique d'haplodiploïdisation entre le stade de croissance de 5 à 9 selon l'échelle de Feekes modifiée par Large;

b) après pollinisation par un pollen fertile, provenant de plantes non traitées situées dans l'environnement des plantes traitées on trie les caryopyses anormaux parmi tous les caryopses obtenus;

c) on régénère les plantes à partir des embryons des caryopses anormaux; et

d) on réalise l'autofécondation de chacune des plantes régénérées pour obtenir les semences correspondant, dans certains cas, à des plantes homozygotes dans la génération suivante.

Le traitement est effectué, de préférence, aux stades 6, 7 ou 8 de l'échelle de Feekes.

Parmi les céréales qui doivent être traitées, il faut citer notamment: le blé dur, le blé tendre, l'orge, le triticale, l'avoine, le sarrasin, le maïs, le riz, le sorgho et le seigle.

Les agents chimiques d'haplodiploïdisation sont des composés chimiques susceptibles de conduire à la formation de caryopses anormaux haploïdes.

Parmi ces agents d'haplodiploïdisation, il faut citer les agents chimiques d'hybridation (A.C.H.), les gamétocides, les composés agissant comme substance de croissance.

Les agents induisant, en outre, une stérilité mâle sont les plus intéressants lors de l'utilisation ultérieure des produits végétaux obtenus.

Parmi les agents chimiques utilisables, il faut citer les composés objet des brevets:

FR—2 352 801
US—3 576 814
US—3 503 986
US—3 761 240
FR—2 383 605
US—4 028 084
US—4 038 065
US—4 238 220
US—4 147 528
US—4 051 142
US—4 345 934
EP—83301057

qui sont notamment des oxo et thio-pyridazines, des pyrimidines ou des oxonicotinates.

Mais il faut citer également d'autres agents à action gamétocide tels que l'acide gibberellique, le TIBA, l'oestrone, les méthanopyrolidines du brevet DE—A—2 641 295 ou les dérivés de l'acide cinchoninique décrits au brevet US—A—4 009 020, ainsi que les composés mentionnés dans l'article de J. J. Batch, Monogr. Br. Crop. Proc. Counc., 1978, 21, 33—43.

Il faut citer également l'acide 2-chloroéthylphosphonique et ses sels et dérivés, ainsi que le chlormequat et ses dérivés et sels.

Parmi les agents précédents, il faut citer, en particulier, les composés de formule:

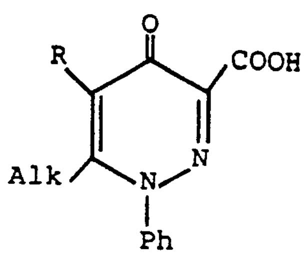

dans laquelle Ph représente un radical phényle non substitué ou substitué, par exemple par un ou plusieurs atomes d'halogène; Alk est un radical alkyle, de préférence en $C_1$ à $C_5$; R est un radical alkyle en $C_1$ à $C_5$, un halogène ou un atome d'hydrogène; et leurs sels.

Ces composés peuvent être préparés par des procédés décrits aux brevets FR—A—2 392 980 et FR—A—2 383 605.

Il s'agit plus particulièrement du composé de l'acide 1-(4-chlorophényle)-1,4-dihydro-4-oxo-6-méthylpyridazine-3-carboxylique et ses sels, en particulier le sel de potassium.

L'époque de traitement ainsi que le dosage des A.C.H. pourront varier suivant la nature de l'A.C.H. et de la céréale traitée, ainsi pour le blé le traitement sera effectué de préférence avec une dose de l'ordre de 0,04 à 10 kg/ha de principe actif, et de préférence à une dose de 0,1 à 2 kg/ha.

L'agent chimique d'haplodiploïdisation peut être appliqué sous une forme quelconque utilisable en agriculture, le principe actif étant formulé avec un support acceptable, par exemple sous forme d'une pulvérisation de poudre ou de liquide sur les plantes.

Une caractéristique importante du procédé est le fait que les plantes traitées doivent être pollinisées par un pollen fertile, et pour cela, présenter dans leur voisinage des plantes non traitées qui fourniront le pollen.

Les caryopses anormaux sont nettement visibles, en particulier sur le blé car il présente un aspect plat et fripé; ces caryopses anormaux présentent, en général, un albumen réduit et possèdent un embryon, dont le point de départ est une cellule haploïde.

De préférence, les plantes sont régénérées par culture *in vitro* de l'embryon prélevé des caryopses anormaux sur un milieu de régénération tel que $R_9$. Les plantes peuvent être régénérées également par germination des caryopses anormaux et culture desdites germes.

Les milieux de culture *in vitro* pour les embryons sont connus de l'homme de métier et ne constituent pas une caractéristique de la présente invention.

Les plantes obtenues sont autofécondées par des procédés connus tels que l'ensachage des épis afin d'obtenir des semances correspondant à des plantes homozygotes.

Les lignées pures fixées ainsi obtenues sont utiles comme outil de recherche, mais également comme parents d'hybrides.

Les exemples ci-après sont destinés à illustrer d'autres caractéristiques et avantages du procédé selon la présente invention.

Exemple 1

Le matériel était constitué de 240 lignées F'3 de blé tendre d'hiver installées au champ obtenues après deux années de sélection dans un programme de sélection récurrente multilocale.

La variabilité génétique intra-lignées et inter-lignées de ces F'3 était très importante car la population servant de matériel de base provenait d'un croisement pyramidal à 16 parents.

Ces lignées devant être intercroisées entre elles, ce qui constitue une opération normale de la fin d'un cycle de sélection récurrente, il a semblé intéressant de traiter une partie de ces lignées avec le produit A.C.H. n° 1 afin de rendre mâle-stérile une partie des plantes de chaque lignée ce qui devait rendre de grands services pour faciliter le croisement.

C'est donc la moitié de chaque ligne de 11 plantes qui a été traitée en avril 1981, toutes les lignes ayant été traitées le même jour à un stade repéré comme étant en moyenne proche du stade optimum tel que cela est notamment décrit dans le brevet français ROHM & HAAS n° 2 383 605.

Dans le cas présent, les plantes ont été traitées avec 1 kg/ha d'une formulation de 1-(4-chlorophényl)-4-oxo-6-méthylpyridazinone-carboxylate de potassium. Au moment de l'application, les plantes se trouvaient à des stades de croissance compris entre 5 et 9 (échelle de Feekes), le stade de développement étant en moyenne de 6 à 7.

On a utilisé à titre d'agent tensio-actif du triton X-100 à 0,03% et le composant actif a été utilisé dans un volume porteur de 500 l/ha.

Il est cependant important de remarquer que compte tenu de la variabilité génétique des lignées traitées, il est évident qu'une partie des plantes ont été traitées à un stade plus ou moins éloigné du stade optimum d'application pour obtenir des mâles-stériles.

Les fleurs des épis des plantes traitées ont été observées *in situ* pendant les semaines qui ont suivi la période de pollinisation.

On a pu observer après 2 à 4 semaines suivant cette période un grand nombre d'épis portant des caryopses anormaux. La principale caractéristique de ces caryopses était qu'ils étaient dépourvus d'albumen et apparaissaient donc comme des caryopses plats. Leur aspect était assez semblable à des caryopses plats que l'on obtient lorsque l'on croise, par exemple, *Hordeum vulgare* par *Hordeum bulbosum*. Ces caryopses pouvaient présenter presque la longueur du grain normal résultant de la double fécondation.

La fréquence de ce phénomène a été notée et s'est montrée très différente d'une plante à l'autre comme cela est indiqué dans le tableau 1. Ainsi, sur 15 plantes différentes, un total de 234 caryopses anormaux a été dénombré pour 93 épis observés avec de 4 à 9 épis comptés par plante. Le nombre moyen de caryopses anormaux variait de 0,8 à 8,50 part épi et par plante.

On a ensuite pratiqué la dissection de ces caryopses anormaux prélevés quelques semaines après la fécondation.

Cette dissection a montré que ces caryopses étaient réellement dépourvus d'albumen normal, celui-ci n'apparaissant que sous la forme d'un liquide plus ou moins blanc, mais par contre ces caryopses possédaient un embryon visible à sa place normale. Ces embryons paraissaient plus petits que les embyrons issus de grains normaux avec une diversité dans l'aspect morphologique de leur scutellum.

Sur certains des caryopses il n'a pas été trouvé d'embryon, soit parce qu'ils étaient absents, soit parce que tropu petits ils ont été perdus dans l'albumen liquide.

Afin d'effectuer une culture *in vitro* des plantes à partir de ces embryons, on a procédé de la façon suivante:

Les caryopses ont d'abord été désinfectés suivant le procédé habituel:

lavage pendant 1 minute dans une solution de teepol à 2%,

désinfection dans une solution d'hypochlorite de calcium à 4% pendant 3 minutes,

rinçage à l'eau stérile deux fois.

Les embryons, une fois disséqués et sortis de 1 enveloppe des grains, ont été transportés sur un milieu de régénération R9 ayant la composition suivante:

Macro-éléments et micro-éléments de Miller

Vitamines de Fuji
Fer EDTA $10^{-4}$ M
A.I.A. $10^{-6}$
Saccharose 2%
Agar 0,8%
pH 5,9.

Il s'agit là d'un milieu couramment employé pour régénérer les plantes haploïdes à partir des embryons androgénétiques obtenus en culture d'anthères in vitro.

Après 3 semaines, 74 des 88 embryons mis en culture ont donné naissance à des plantules chlorophyliennes complètes et qui ressemblaient de façon très frappante à des plantes haploïdes.

Leur croissance était vigoureuse et leur enracinement in vitro était normal.

Afin de connaître le niveau de ploïdie des plantules, on a prélevé des pointes de racines suivant le procédé habituel (préfixation dans une solution d'alpha-monobromonaphtalène à 2%, fixation dans l'alcool à 70°, coloration au carmin acétique).

2 à 3 pointes de racine par plante ont été comptées pour 15 plantes.

Ce comptage a permis de découvrir que le niveau de ploïdie à l'intérieur d'une même racine, et ceci pour toutes les plantes, variait de l'état haploïde à l'état diploïde.

D'une plaque métaphasique à l'autre dans une même racine, on a constaté que le nombre des chromosomes variait de l'état haploïde $n = 21$ à l'état diploïde $n = 42$ avec des niveaux intermédiaires: 21—27, 28, 29—33, 35, 36—41, 42.

Ce résultat pourrait signifier que la cellule initiale de l'embryon était haploïde et qu'au cours des divisions cellulaires de ces embryone le niveau de ploïdie est devenu instable, peut-être du fait de l'absence d'albumen bien constitué.

Le plus extraordinaire est que le phénomène d'endoreduplication se passait comme si le doublement avait lieu génome par génome:

$$ABD \rightarrow AABD \rightarrow AABBD \rightarrow AABBDD$$

Il n'est pour l'instant pas possible de se prononcer sur l'orogine de cette cellule haploïde; il pourrait s'agir d'une parthénogénèse induite ou bien d'une androgénèse in situ.

Cependant, l'origine étant certainement haploïde et une endoreduplication se manifestant dans ces plantes, celles-ci devaient à ce moment là donner lieu à une descendance haploïde, doublée, homozygote.

C'est ce qui a été démontré dans l'exemple ci-après.

## Exemple 2

Les plantules obtenues par culture d'embryons ont tout d'abord été clonées in vitro, puis vernalisées 8 semaines en tube afin de leur permettre une floraison normale à la sortie en serre.

Seule une partie des plantes a pu être repiquée avec succès en serre et finalement 18 plantes ont pu fleurir normalement.

Comme on pouvait s'y attendre, suivant que le doublement spontané était plus ou moins efficace, les territoires cellulaires des épis étaient mixoploïdes. Ainsi la fertilité variait de 2 à 4 grains par épi jusqu'à une fertilité normale. 2 épis par plante avaient été ensachés pour vérifier ce point.

Afin de vérifier que les grains récoltés sur chaque plante avaient bien tous la même constitution génétique, résultat attendu si les plantes issues des caryopses anormaux étaient bien des homozygotes, on s'est intéressés à l'étude des gliadines et des gluténines de 14 grains issus du même épi pour chaque plante.

6 descendances ont ainsi été analysées par électrophorégramme en comparaison avec des descendances F'4 des mêmes lignées F'3 d'origine, obtenues par autofécondation.

Pour 4 descendances des caryopses anormaux, les 14 électrophorégrammes de gliadines se sont montrés rigoureusement identiques entre eux.

Pour une descendance, seul un grain différait seulement par une bande des 13 autres identiques entre eux.

Pour le dernier cas, 7 grains présentaient un spectre identique, les 7 grains présentaient un spectre identique au premier avec 2 bandes en moins.

Par contre, pour les gluténines, la stabilité était parfaite.

En comparaison, les descendances F'4 présentaient pour la plupart de nombreuses différences dans leur bande de gliadines.

Il ressort des exemples précédentes que les plantes obtenues à partir des caryopses anormaux sont des plantes haploïdes à caractère mixoploïde pouvant alors donner lieu à une descendance homozygote.

Ceci ouvre une nouvelle voie totalement originale d'haplodiploïisation du blé tendre sur des plantes ayant subi un traitement par un A.C.H.

TABLEAU 1

| N° de la plante | Nombre d'épis observés | Nombre de caryopses anormaux | Nombre moyen par épi |
|---|---|---|---|
| 1 | 4 | 29 | 7,25 |
| 2 | 4 | 34 | 8,50 |
| 3 | 6 | 6 | 1 |
| 4 | 6 | 19 | 3,17 |
| 5 | 5 | 5 | 1 |
| 6 | 4 | 5 | 1,25 |
| 7 | 6 | 13 | 2,17 |
| 8 | 6 | 22 | 3,67 |
| 9 | 7 | 18 | 2,57 |
| 10 | 6 | 5 | 0,83 |
| 11 | 6 | 10 | 1,67 |
| 12 | 6 | 15 | 2,50 |
| 13 | 9 | 23 | 2,56 |
| 14 | 9 | 17 | 1,89 |
| 15 | 9 | 13 | 1,44 |
| Totaux | 93 | 234 | 2,52 |

Exemple 3

Etudes sur l'Application de la Technique aux Champs

Détermination du meilleur stade et de la meilleure dose de traitement

Sauf indication contraire, le traitement est effectué comme dans l'exemple 1.

L'objectif est de déterminer les "doses stades" les plus efficaces pour l'apparition du phénomène "grains plats" et de voir s'il y a une liaison ou pas entre ce phénomène et le taux de stérilité mâle obtenu.

Le produit, en solution aqueuse et additionné d'un mouillant, est pulvérisé sur la totalité de la plante à traiter.

En 1983, deux doses du produit ont été testées, $d_1$ et $d_2$, et en 1984, trois doses, $d_1$, $d_2$ et $d_3$; $d_1 = 1{,}0$ kg/ha; $d_2 = 1{,}4$ kg/ha et $d_3 = 1{,}8$ kg/ha.

A chaque traitement, des échantillons ont été prélevés pour déterminer le stade de développement atteint par les plantes.

En 1983, on a travaillé, aux champs, sur un seul dispositif expérimental, comprenant 4 variétés et 2 hybrides $F_1$.

Codes: A = Frandoc, B = Festin
$F_1$ A × B = Frandoc x Festin
C = Courtôt, D = Fidel
$F_1$ C × D = Courtôt × Fidel.

Deux stades ont été testés, codés $t_2$ et $t_3$.

Stade $t_2$: traitement le 3 mai 1983. Les variétés Fidel et Frandoc avaient le 2ème noeud visible; la dissection de quelques plantes prélevées au hasard dans l'essai montrait que l'épi était en moyenne à 0,7 cm du plateau de tallage.

Au stade $t_3$, le 9 mai, Frandoc avait atteint le stade 8 de l'échelle de Feekes modifiée par Large (dernière feuille pointante), l'épi était à 1,5 cm du plateau de tallage, Fidel, plus tardif, n'avait pas encore la dernière feuille pointante et sans épi était à 1 cm du plateau de tallage.

L'échelle de Feekes modifiée par Large est donnée à la fin de la description.

Le tableau 2 montre le nombre moyen de grains plats par épi pour les deux $F_1$ A × B et C × D.

TABLEAU 2

Annee 1983 — Nombre Moyen de Grains Anormaux par EPI (Moyenne de Deux Repetitions par Dose-Temps)

Effectif d'épis par dose-temps et répétition: 20 sauf pour $d_2t_3$: 40

$F_1$ A × B

| | $T_2$ | $T_3$ |
|---|---|---|
| $d_1$ | 1,05 | 0,575 |
| $d_2$ | 0,8 | 2,03 |

$F_1$ C × D

| | $T_2$ | $T_3$ |
|---|---|---|
| $d_1$ | 0,8 | 0,7 |
| $d_2$ | 0,5 | 1,31 |

De plus, sur les 4 variétés et les 2 $F_1$, on a mesuré pour 5 épis ensachés la fertilité exprimée en nombre de grains normaux par épillet.

Les résultats sont donnés au tableau 3.

Pour cet essai, on a étudié plus particulièrement les deux hybrides $F_1$ pour trois raisons:

Pour l'aspect production d'haploïdes doublés agronomiquement intéressants, il est préférable de travailler un matériel hybride.

Le tri des haploïdes doublés (HD) obtenus par cette méthode est beaucoup plus aisé à partir d'un matériel hétérozygote qu'à partir de lignées. En effet, un pourcentage d'erreur existe et le dépistage précoce des diploïdes issus d'autofécondations ou d'hybridations n'est pas aisé par comptage chromosomique.

En ce qui concerne l'essai dose-temps, la première constatation est que la fréquence d'apparition du phénomène est faible pour l'année 1983: la valeur maximum de 2,03 grains plats/épi est inférieure aux premières observations faites depuis 1981.

En effet, tous les battages depuis 1981 montrent une fréquence proche de 3 grains plats per épi.

Au temps $t_2$, les doses $d_1$ et $d_2$ ne semblent pas très discriminantes, les différences observées sont faibles et les fréquences les plus élevées sont trouvées pour la dose $d_1$, la plus faible. Au temps $t_3$, par contre, on constate pour les deux $F_1$ une nette augmentation de la fréquence pour la dose $d_2$.

Il semble donc que le traitement soit le plus efficace à un stade relativement avancé et à une dose importante ($d_2$ $t_3$). Toutefois, il n'est pas utile apparamment d'augmenter la dose de traitement à un stade trop jeune.

Le tableau 3 concernant la fertilité fait apparaître plusieurs points intéressants:

Pour tous les génotypes testés, c'est effectivement la combinaison stade-dose $d_2$ $t_3$ qui produit le plus grand taux de stérilité.

Pour la série A, B et A × B, on se rend compte que le stade du traitement a été très important puisque c'est toujours au stade $t_3$ que la stérilité est la plus forte par rapport au stade $t_2$. A $t_2$, la dose employée n'est pas très discriminante, sauf pour B. A $t_3$ par contre, c'est la dose $d_2$ la plus efficace.

Pour la série C, D et C × D, le problème est différent et on trouve un fort taux de stérilité seulement à $d_2$ $t_3$ pour D; $d_1$, $t_3$, $d_2$, $t_3$, $d_1$ $t_2$ ne se démarquent pas vraiment les uns des autres d'une façon claire. A cela deux explications sont possibles:

Les 6 génotypes ont été traités aux mêmes dates, or la série C, D, C × D est un peu plus tardive que la série A, B, X × B.

Il se peut donc que le stade ait été moins favorable et l'essai moins discriminant.

Il ne faut pas omettre la possibilité d'une interaction traitement-génotype, que ce soit au niveau de la stérilité induite par le traitement ou au niveau du phénomène "grains plats".

Détermination du meilleur stade et de la meilleure dose pour un autre génotype (la variété Festival)

Pour cet essai, on a utilisé des parcelles expérimentales de 6 m2 des variétés Festival et Fidel implantées aux champs. Ces parcelles avaient été semées à la dose de 250 grains germant au m2 à l'automne 1983.

TABLEAU 3

Fertilite, Apres Traitement Gametocide, Mesuree pour Cinq Epis Ensaches, pour Quatre Varietes et Leurs Deux Hybrides $F_1$

(La fertilité est exprimée en nombre de grains "normaux" par épillet)

A

| | $T_2$ | $T_3$ |
|---|---|---|
| $d_1$ | 2,56 | 1,2 |
| $d_2$ | 2,8 | 0,07 |

B

| | $T_2$ | $T_3$ |
|---|---|---|
| $d_1$ | 2,18 | 0,3 |
| $d_2$ | 1,08 | 0,04 |

A × B

| | $T_2$ | $T_3$ |
|---|---|---|
| $d_1$ | 3 | 0,64 |
| $d_2$ | 2,09 | 0,42 |

C

| | $T_2$ | $T_3$ |
|---|---|---|
| $d_1$ | 1,82 | 1,22 |
| $d_2$ | 0,85 | 0,84 |

D

| | $T_2$ | $T_3$ |
|---|---|---|
| $d_1$ | 2 | 1,45 |
| $d_2$ | 1,45 | 0,2 |

C × D

| | $T_2$ | $T_3$ |
|---|---|---|
| $d_1$ | 1,7 | 2,23 |
| $d_2$ | 2,9* | 2,06 |

* un seul épi

Le protocole expérimental a été le suivant: 3 doses ($d_1$, $d_2$, $d_3$) ont été testées à trois stades différents ($t_1$, $t_2$, $t_3$). Chaque parcelle expérimentale était constituée par 1/4 de parcelle d'essai; chaque dose-temps a été répétée 4 fois au hasard parmi les parcelles traitées, un nombre égal de parcelles témoins.

Les dates de traitement ont été les suivantes:

stade $t_1$ : 25 avril 1984
stade $t_2$ : 30 avril 1984
stade $t_3$ : 4 mai 1984.

Cet essai a été dépouillé de la manière suivante:

Sur chaque dose-temps, en fécondation libre, on calcule la fréquence de grains plats par épi, sur un effectif total de 20 à 40 épis. Ce même résultat est exprimé en indice de fertilité (%) en grains plats:

$$\text{indice de fertilité (\%)} = \frac{Ft}{Fc} \times 100$$

Ft = Nb grains plats/épillet sur plantes traitées
Fc = Nb grains normaux/épillet sur témoin (non traité).

Les parcelles témoins ont permis de mesurer la fertilité hors traitement, en autofécondation et en fécondation libre.

Sur les parcelles traitées, des sachets d'autofécondation ont été utilisés; la stérilité induite par chaque traitement en pourcentage du témoin est calculée suivant la formule:

$$\text{stérilité (\%)} = \frac{Sc - St}{Sc} \times 100$$

Sc = Nb grains/épillet des autofécondations sur plantes témoins
St = Nb grains/épillet des autofécondations sur plantes traitées.

Pour le calcul de St, les trois types de grains ont été compatibilisés (grains normaux, échaudés, plats).

Les résultats sont synthétisés dans le tableau 4.

TABLEAU 4

Annee 1984 — Essai Dose-Temps sur la Variete Festival

Résultats sur plantes traitées en fécondation libre:
A — Exprimés en nombre moyen de grains plats par épi.
B — Exprimés en indice de fertilité (%) en grains plats.

Effectif d'épis analysés par dose-temps: 10 (sauf $d_3$ : 5)

A

| | dose \ temps | $T_1$ | $T_2$ | $T_3$ |
|---|---|---|---|---|
| 1 | $d_1$ | 0,45 | 0,87 | 2,95 |
| 1,4 | $d_2$ | 0,8 | 1,82 | 3,3 |
| 1,8 | $d_3$ | 0,85 | 2,5 | 3 |

B

| | dose \ temps | $T_1$ | $T_2$ | $T_3$ |
|---|---|---|---|---|
| 1 | $d_1$ | 1,33% | 2,56% | 8,55% |
| 1,4 | $d_2$ | 2,37% | 5,4% | 9,62% |
| 1,8 | $d_3$ | 2,52% | 7,42% | 8,87% |

En ce qui concerne la fréquence de grains plats par épi, on constate d'abord:

qu'il existe des nettes différences selon les doses-temps (7,5 fois plus de grains plats en $d_2$ $t_3$ qu'en $d_1$ $t_1$);

pour chaque dose, c'est le traitement le plus tardif qui a été le plus efficace;

si par contre on s'intéresse, pour un stade donné, à l'opportunité d'augmenter la dose, on s'aperçoit que cela semble plus efficace aux stades les plus précoces (peut-être $t_1$ mais surtout $t_2$); par contre, au stade $t_3$, la dose n'a qu'une relative importance; la fréquence la plus élevée est observée en $d_2$ $t_3$, en $d_3$ $t_3$, cette fréquence baisse comme si on avait atteint un seuil d'efficacité à la dose $d_2$.

Ce tableau montre clairement qu'il est très important de bien isoler le stade de traitement qui ne doit pas être trop précoce. Si le stade est bien choisi, il n'est pas utile de traiter à des doses élevées.

L'ensemble des résultats 1983 et 1984 sont donc cohérents sur un certain nombre de points:

Importance de traiter à un stade assez tardif, proche du stade 8 de l'échelle de Feekes.

Si le stade de traitement est correct, une dose moyenne suffit (dose $d_2$).

En ce qui concerne la stérilité induite par le traitement, elle est totale pour les doses-temps $d_2$ $t_3$, $d_3$ $t_3$ et $d_3$ $t_2$, très forte pour $d_2$ $t_2$ et $d_1$ $t_3$. Là encore, le stade de traitement semble le plus important et le stade le plus tardif le plus efficace. La liaison stérilité induite-apparition de grains plats paraît bonne, si on compare les résultats, et les deux phénomènes paraissent liés. Le coefficient de corrélation entre les deux est de 0,8, ce qui indique effectivement une liaison positive.

En ce qui concerne la correspondance entre le stade atteint au moment du traitement par la plante traitée et l'efficacité du traitement, un certain nombre d'épis ont été disséqués aux dates de traitement et on a observé le développement des pièces florales. C'est l'épi du maître brin qui a toujours été observé.

Dans le cas de l'essai Festival, il semble que le traitement est le plus efficace au stade $C_2$, c'est-à-dire quand les anthères ont fini leur différenciation.

Il faut remarquer que l'ovaire se différencie plus tardivement : au stade $C_2$ le stigmate est en train de se former.

Exemple 4

Role du Pollen dans le Denclenchement du Phenomene "Grains Plats"

Afin d'élucider le rôle de la qualité du pollen et de l'état de l'ovaire dans le déclenchement du phénomène "grains plats", on a mis en place en 1984 une expérience de castration pollinisation contrôlée de plantes traitées par le gamétocide.

Des plantes de la variété Festival non traitées (témoins) et des plantes traitées à une seule dose mais à deux stades différents ($d_2$ $t_2$ et $d_2$ $t_3$) ont été castrées puis pollinisées avec des pollens différents:

Par du pollen viable, issu de plantes non gamétocidées; ce pollen était soit de l'autopollen, soit de l'allopollen provenant de la variété Fidel;

Par du pollen provenant de plantes traitées par le gamétocide, auto et allo comme dans le cas précédent.

La pollinisation a été faite par 3 épis mâles pour chaque épi castré, de la même façon pour tous les cas de figure. Ajoutons que ce que l'on sait de l'action du gamétocide fait penser qu'il n'y a pas ou très peu d'émission de pollen chez les plantes traitées. Mais on souhaiterait savoir si un reliquat de pollen plus ou moins viable ne pouvait pas jouer un rôle dans le déclenchement du phénomène.

Enfin, un certain nombre de plantes ont été castrées et n'ont pas été pollinisées, ceci pour éventuellement mettre en évidence une parthénogénès induite par le traitement gamétocide et en absence de pollen.

Les résultats de cette expérience sont donnés au tableau 5. Chaque case de ce tableau représente une valeur moyenne de deux répétitions.

TABLEAU 5

Valeur moyenne de la frequence de formation de grains plats par fleur castree ches la variete festival

| traitement des plantes / type de pollinisation | plants* non traitées | traitement $D_2$ $T_2$ | traitement $D_2$ $T_3$ |
|---|---|---|---|
| Pas de pollen | 0,000 | 0,000 | 0,000 |
| Pollinisation Autopollen Gamétocidé | 0,000 | 0,067 | 0,000 |
| Pollinisation par Allopollen Gamétocidé | 0,010 | 0,048 | 0,082 |
| Pollinisation par Autopollen non Gamétocidé | 0,270 | 0,276 | 0,528 |
| Pollinisation par Allopollen non Gamétocidé | — | 0,239 | 0,373 |
| Autofécondations** | 0,626 | 0,014 | 0,005 |

* Pour les plantes non traitées, les fréquences de grains sont celles de grains normaux; aucun grain plat n'a été observé.

**Pour les autoféondations, il s'agit de la fréquence de grains par fleur à partir d'une estimation de 3 fleurs fertiles par épillet.

Les résultats paraissent relativement clairs et intéressants:

En ce qui concerne les épis non pollinisés, aucun grain n'a été trouvé, ce qui montre que le traitement gamétocide, en l'absence de pollen, n'induit pas la parthénogénèse d'une façon détectable.

les autofécondations étaient des témoins de la fertilité mâle et femelle pour les plantes traitées. Dans le cas des témoins, elles permettent d'avoir une idée de "l'effet castration"; en effet, en autofécondation on a 0,626 graines par fleur fertile en moyenne et quand on castre puis pollinise avec de l'autopollen, 0,270 grains par fleur castrée. Le choc castration aurait donc réduit d'environ un facteur 2 la probabilité de formation d'un grain.

Bien sûr, ce calcul est approximatif, mais il semble important d'avoir une idée sur cette question pour estimer la fiabilité des résultats, un résultat négatif pouvant être dû aussi à un problème technique; ce n'est apparamment pas le cas pour cette manipulation.

Les résultats des autres cas de figure permettent de dégager plusieurs points:

Les différences importantes qui existent entre fréquence d'apparition de grains plats selon la qualité du pollen (gamétocidé ou pas) indiquent que la présence de pollen viable provenent de plantes non traitées est indispensable au déclenchement du phénomène.

Que ce pollen soit de l'auto ou de l'allopollen, n'a apparamment pas d'incidence du moins en première approche.

Les faibles quantités de grains plats observées dans le cas de pollinisation par du pollen gamétocidé sont probablement dues à des reliquats de pollen fertile. Cette hypothèse est cohérente avec les résultats obtenus en ce qui concerne l'autopollen gamétocidé: il n'y a aucun grain plat obtenu pour $d_2$ $t_3$, où le taux de stérilité est de 100%, par contre une faible fréquence pour $d_2$ $t_2$, où le taux de stérilité était légèrement plus faible (99,1%).

Enfin, il semble qu'on obtienne plus de grains plats au temps-dose $d_2$ $t_3$ (pollinisation avec du pollen non gamétocide), ce qui laisse supposer que le produit a aussi une action sur l'ovaire, action plus ou moins marqué selon le strade de traitement.

Ainsi, c'est le pollen des plantes non traitées sur un ovaire de plante traitée qui déclenche le phénomène apparition de grains plats.

## Exemple 5

### Obtention d'haploides par mise en culture d'embryons taux de regeneration

En juillet, au cours de la maturation du grain, on peut observer des symptomes de développement anormal ou d'avortement sur certains grains des plantes traitées.

Le grain est à cette date au stade laiteux (11—1 sur l'échelle de Feekes). Les grains des plantes témoins sont verts, lisses; l'albumen est abondant, de couleur laiteuse. Le caryopse peut être disséqué aisément, et l'embryon a un aspect normal d'embryon immature.

Chez certains grains des plantes traitées, on observe un début de recroquevillement du grain, il se ride, prend parfois une couleur sombre. Si on dissèque le caryopse, on se rend compte que l'albumen est en train de dégénérer, l'embryon présente deux caractéristiques:

Souvent il est totalement anormal, de taille variable, avec le scutellum de forme extravagante; parfois on a du mal même à reconnaître les différents organes d'un embryon, mais on set trouve devant une masse cellulaire apparamment indifférenciée.

Parfois il a une apparence normale, mais il est de taille extrèmement petite par rapport à un embryon témoin.

Ces embryons jugés "anormaux" prélevés sur des plantes traitées et dont les grains présentaient des signes de dégénérascence de l'albumen ont été mis en culture.

En 1981, au cours de la première manipulation, 88 embryons transplantés sur milieu de régénération $R_9$ avaient régénéré 74 plantules. Après clonage et vernalisation 18 plantes fertiles avaient été obtenues, soit:

taux de régénération: 84%
taux de survie: 24%
(dû à des conditions stressantes de serre).

En 1983, compte tenu des difficultés à observer le phénomène, 38 embyons seulement ont été mis en culture, et 20 d'entre eux ont régénéré en plantes chlorophyliennes, après sortie du tube vernalisation et repiquage en serre, 15 ont survécu, ce qui donne:

taux de régénération: 52%
taux de survie: 75%.

L'origine de ces plantes est la suivante: $F_1$ C × D: 7; $F_1$ A × B: 4; A: 3; D: 1.

En 1984, les cultures d'embryons ont concerné le blé, mais également l'orge et le tricicole.

Pour le blé, 189 embryons ont été mis en culture; 41 ont régénéré (soit 21,7%) et 36 plantes ont survécu à cette date (soit 87,8%).

On peut remarquer que le taux de régénération est particulièrement faible en 1984. Dans ce cas, un seul rinçage à l'eau stérile après l'hypochlorite de calcium a été fait, ce qui peut expliquer ce faible taux. Par contre, que ce soit en 1983, en 1984, on perd peu de plantes après la régénération, c'est-à-dire à la sortie de tube ou en vernalisation.

Exemple 6

Obtention d'haploïdes par germination des grains plats

L'autre voie pour obtenir des haploïdes à partir des plantes traitées consiste à attendre la maturité puis à battre délicatement les épis récoltés. Si l'on prend soin, après le battage, de ne pas aspirer les glumes et les glumelles et qu'on trie soigneusement, on trouve des "grains plats". Ce sont des grains petits, ridés, presque sans albumen, parfois à peine identifiables comme grains.

Si on met à germer ces grains, on s'aperçoit avec surprise qu'un certain nombre d'entre eux germent. Il ne reste plus qu'à suivre les plantules obtenues par germination après avoir prélevé leurs racines très précocement (pour comptages chromosomiques).

Cette technique comme on le voit a le mérite de la simplicité et évite le passage *in vitro*. Cependant, là aussi, se pose le problème du crible choisi dans le tri des grains plats: c'est un crible uniquement visuel et il n'est pas toujours très objectif. Par ailleurs, le tri des grains plats est long et fastidieux.

Les tableaux 6 et 7 résument les résultats de deux manipulations réalisées en 1983. Le tableau 6 montre sur 19 $F_3$ un nombre élevé de grains plats par épi, proche de 3,5: 38% de ces grains germent.

Les résultats du tableau 7 sont concordants: 40% des grains plats issus de A, B, C, D, et de leurs hybrides $F_1$, sont capables de germer. Sur 100 grains qui germent, 72 plantules survivent après vernalisation et repiquage en serre.

Sur la base de tous ces résultats, un calcul rapide montre l'intérêt de la méthode: un traitement bien effectué, au bon stade, peut donner 3 à 3,5 grains plats par épi: compte tenu du pourcentage de germination et de survie, on peut espérer régénérer, dans les meilleurs cas, une plantule supposée haploïde par épi traité.

Ce rendement semble relativement bon comparé par exemple à celui de l'androgénèse *in vitro* où on obtient 1 à 2 haploîdes doublés pour 1 000 anthères mises en culture (soit environ 15 épis).

TABLEAU 6

Annee 1983 — Resultats du battage de 19 $F'_3$ cinq epis ont ete battus par origine

Fertilité: nombre de graines par épillet

| Origine | Fertilite | Nombre de grains plats/EPI | % Germination |
|---|---|---|---|
| $BCF_4\ pl_{7-5}$ | 2,55 | 2 | 40 |
| $BCF_7\ l_2\ pl_{4-3}$ | 2,72 | 2,6 | 23,1 |
| $DCF_5\ pl_{7-3}$ | 1,74 | 9,8 | 36,7 |
| $DCF_{11}\ l_2\ pl_{1-2}$ | 0,28 | 6,2 | 41,9 |
| $DR_9\ pl_{4-4}$ | 0,25 | 3,2 | 31,25 |
| $ECF_3\ pl_{8-5}$ | 3,28 | 4,8 | 8,33 |
| $ECF_4\ pl_{9-3}$ | 2,25 | 2,4 | 41,7 |
| $ECF_5\ pl_{8-4}$ | 0,58 | 2,2 | 27,3 |
| $ER_7\ l_1\ pl_{5-4}$ | 0,25 | 3,8 | 57,9 |
| $FCF_1\ l_1\ pl_{5-5}$ | 1,84 | 1,4 | 0 |
| $FCF_6\ l_1\ pl_{4-5}$ | 1,72 | 2,4 | 50 |
| $FCF_8\ l_1\ pl_{4-5}$ | 0,7 | 2,6 | 69,2 |
| $FeF_{13}\ pl_{4-2}$ | 0,31 | 0,8 | 50 |
| $FR_8\ l_1\ pl_{1-1}$ | 2,8 | 2 | 21,4 |
| $FR_8\ l_1\ pl_{1-2}$ | 2,6 | 6,2 | 32,3 |
| $GR_2\ l_1\ pl_{1-1}$ | 1,94 | 6,8 | 50 |
| $GR_2\ l_2\ pl_{4-4}$ | 1,3 | 1,6 | 50 |
| $GR_4\ pl_{1-5}$ | 0,4 | 1,8 | 33,3 |
| $GR_5\ pl_{7-5}$ | 1,81 | 2 | 70 |
| Moyenne | | 3,44 | 38,65 |

TABLEAU 7
Resultats de la germination de grains plats (Recolte 1983)

| Origine | Effectif | Effective Germe | % Germination % | De Survie |
|---|---|---|---|---|
| A | 26 | 10 | 38,5 | 70 |
| B | 52 | 16 | 30,8 | 37,5 |
| C | 31 | 8 | 25,8 | 12,5 |
| D | 30 | 20 | 66,7 | 65 |
| $F_1$ C × D | | | | |
| $d_1$ $t_2$ | 12 | 9 | 75 | |
| $d_1$ $t_3$ | 11 | 5 | 45,5 | |
| $d_2$ $t_3$ | 11 | 4 | 36,4 | |
| $d_3$ $t_3$ | 22 | 6 | 18,2 | |
| Total | 56 | 24 | 39,3 | 92 |
| $F_1$ A × B | | | | |
| $d_1$ $t_2$ | 12 | 8 | 58,3 | |
| $d_1$ $t_3$ | 11 | 5 | 45,5 | |
| $d_2$ $t_2$ | 9 | 5 | 44,4 | |
| $d_2$ $t_3$ | 19 | 9 | 42,1 | |
| Total | 51 | 27 | 47,1 | 85 |
| | 246 | 100 | 40,65 | 72 |

Exemple 7
Comptages Chromosomiques

Les comptages chromosiques effectués sur les premières plantes obtenues par cette méthode avaient montré un phénomène de mixoploïdie (exemple 2) caractérisé par des plaques métaphasiques de différents niveaux de ploïdie à l'intérieur de chaque racine observée. Ces niveaux variaient de 21 à 42, avec notamment des intermédiaires entre les deux. Il faut rappeler que ces premières plantes étaient issues de culture *in vitro*.

Au course printemps 1983, on a compté un grand nobre de pointes de racines prélevées très précocement, sur des germinations de grains plats, pour voir si on observe le même phénomène. La technique de coloration utilisée est celle de Feulgen.

Il en résulte que:

la majorité des plaques comptées comportaient 42 chromosomes, ce qui indique que le doublement commencerait très précocement;

on a observé plusieurs fois, très clairement, des plaques à 21 chromosomes, ce qui montre qu'il y a bien un passage par une phase haploïde;

beaucoup d'intermédiaires entre ces deux extrèmes sont présents, avec notamment des groupes autour de 28 et de 35 chromosomes.

Cela laisse supposer que le doublement aurait lieu par génome (soit par unité de 7 chromosomes).

Parmi tous ces comptages, on en cite 3 ci-dessous qui paraissent intéressants. Ils illustrent ce qu'on peut observer par "unité plante" et la nécessité d'observer beaucoup de divisions sur une même plante avant de pouvoir conclure:

| Origine | Nombre de plaques à ce chromosome |
|---|---|
| Fc $F_6$ $I_1$ $pl_{4-5/3}$ | 4 à 42 |
| | 1 à 36 |
| | 1 à 28 |
| | 1 à 21 |
| $GR_2$ $I_1$ $pl_{1-1/1}$ | 4 à 42 |
| | 1 à 27 |
| | 1 à 21 |
| $DCF_{10}$ $I_1$ $pl_{3-4}$ | 13 à 42 |
| | 1 à 41 |
| | 1 à 21 |

Exemple 8

Observation des plantes aux champs

L'observation des plantes aux champs après une génération de multiplication sous tunnel est aussi un bon critère de jugement qui peut élcairer sur l'origine des plantes obtenues. Il faut rappeler que toutes les premières lignées (codé GAM 1 à 229) sont issues de plantes $F_3$ de la PAO et qui en étaient à leur 3 ème année du premier cycle de sélection récurrente; 80 d'entre elles ont été choisies pour l'intercroisement devant donner la $PA_1$.

On s'est attaché à produire des HD de ces 80 familles en particulier, par la technique décrite ici même.

L'observation de la stabilité aux champs permet théoriquement de discerner les haploïdes doublés issus de ces $F_3$ des lignées qui sont des $F_5$ (donc en disjonction) et qui proviendraient d'une reproduction sexuée normale.

On a observé en 1984 133 "lignées" GMA. Ces plantes étaient semées en semis "Seed matic" sur 3 à 6 lignées selon le nombre de semences disponibles. Chaque lignée GAM est maintenue en autofécondation par ensachage sous tunnel, puis aux champs, de quelques épis; on a semé systématiquement ces lignées issues d'autofécondation à côté des lignées issues de fécondation libre pour pouvoir les comparer.

La stabilité de ces plantes à des moments différents de la végétation a été notée; on a observé surtout les critères morphologiques: taille, glaucescence, port de la dernière feuilile, forme de l'épi, artisation et la précocité (épiaison, floraison). Les autres notations concernant ces plantes ont été faites par l'équipe blé, conjointement aux notations des HD androgénétiques.

Il est apparu nécessaire, à la fin des observations, de nuancer le jugement concernant ces plantes en faisant 4 classes d'homogénéité phénotypique. Les résultats sont donnés au tableau 8.

Au total, environ 37% de ces plantes sont d'emblée en disjonction. 17% sont stables sans aucune réserve, 34,6% avec des réserves dues à des légères différences de taille entre lignées. Au total, environ 52% de plantes sont donc fortement soupçonnées d'être des haploïdes doublés. Pour le reste, le jugement n'était pas possible en 1984.

Les plantes de la classe 4 n'ont pas été récoltées, les plantes des classes 1, 2 et 3 ont été récoltées et resemées en 1985.

Exemple 9

Electrophorese des gliadines

En 1983, on a ainsi observé la stabilité de la première série de GAM, codée de 5 à 50; cette série comporte 13 plantes. Parmi les descendances de ces plantes, 10 étaient homogènes, 3 en disjonction.

TABLEAU 8

Annee 1984 — Resultats de l'observation de 133 lignees gam aux champs pour leur homogeneite

| Classe | Definition | Effectif | % |
|---|---|---|---|
| Classe 1 | Homogène | 23 | 17,3 |
| Classe 2 | Homogène avec réserves* | 46 | 34,6 |
| Classe 3 | Jugement difficile** | 15 | 11,3 |
| Classe 4 | En disjonction | 49 | 36,8 |
| Total | | 133 | 100 |

* Les réserves sont dues à des faibles différences de taille entre lignées, différences probablement dues au terrain

**Jugement difficile à cause d'un nombre de plantes insuffisant

D'autre part, on a réalisé une analyse électrophorétique des gliadines sur les grains issus de ces 13 plantes. Il est intéressant de constater qu'il se trouve 9 plantes pour lesquelles l'analyse ne discerne aucune différence dans les diagrammes des gliadines de plusieurs grains.

Ainsi, 9 plantes trouvent leur place, cette année là, parmi le groupe jugé stable aux champs et ne montrant pas de variabilité lors de l'analyse des gliaidines.

Tous ces arguments: comptages chromosomiques, observations aux champs, électrophorèse des gliadines, montrent que certaines plantes produites par cette technique sont bien des haploïdes doublés.

Exemple 10

Utilisation d'autres substances d'haplodiploidisation pour l'induction du phenomene "grains plats"

En 1983, on a traité les deux $F_1$ A × B et C × D par deux régulateurs de croissance, le Céronne et le Cycocel, utilisés sur céréales à paille. Le Céronne contient de L'Ethérel (acide 2-chloroéthylphosphonique), le Cycocel du chlormequat chlorure ou CCC, qui est un régulateur de croissance.

Seuls une dose et une stade d'application ont pû être testés. Le traitement a été fait alors que les plantes étaient au stade un noeud visible. On a utilisé des doses correspondant au double de la dose préconisée par le fabricant comme régulateur de croissance.

Les résultats sont données au tableau 9.

TABLEAU 9

Nombre moyen de grains plats par empi pour deux $F_1$ traitees par deux regulateurs de croissance et fertilite en autofecondation des deux $F_1$ et de leurs quatre parents

CERONNE

| | | | NB moyen grains Plats/EPI |
|---|---|---|---|
| A | B | $F_1$ A × B | $F_1$ A × B |
| 2,95 | 2,3 | 3,09 | 0,3 |
| C | D | $F_1$ C × D | $F_1$ C × D |
| 1,5 | 1,82 | 1,8 | 0,875 |

TABLEAU 9 (Suite)

CYOCEL

| | | | NB moyen grains Plats/EPI |
|---|---|---|---|
| A | B | $F_1$ A × B | $F_1$ A × B |
| 2,8 | 2,68 | 2,18 | |
| C | D | $F_1$ C × D | $F_1$ C × D |
| 1,91 | 2,14 | 1,96 | 0,6 |

En ce qui concerne la $F_1$ A × B, la fréquence de grains plats est extrêmement faible et on ne dispose que d'une seule valeur. Par contre, pour la $F_1$ C × D, la fréquence de grains plats n'est pas inférieure aux valeurs obtenues pour $d_1$ $t_2$, $d_1$, $t_2$, $d_1$, $t_3$ et $d_2$ $t_2$ avec le gamétocide de l'exemple 1.

Exemple 11

Essais d'application de cette technique a l'orge et au triticale

1) *L'orge*

On traité avec le produit de l'exemple 1, en 1984, 64 $F'_3$ d'orge provenant de Clermont-Ferrand, semés en "Seedmatic". La moitié de chaque lignée a été traitée, l'autre moitié a servi de témoin. Deux doses ont été testées ($d_2$ et $d_3$), chacune à deux stades de développement différents ($t_1$ et $t_2$).

En outre, 7 variétés ont été traitées, ainsi que 6 hybrides $F_1$ suivant le schéma suivant:

variétés: 1 dose, 2 stades testées ($d_2$ $t_1$ et $d_2$ $t_2$)

$F_1$: 1 dose et 2 stades testés ($d_3$ $t_1$ et $d_3$ $t_2$).

Les premiers battages font état de la présence de grains plats à une fréquence faible.

On a obtenu par culture *in vitro* d'embryons immatures deux plants d'orge provenant de deux $F_3$ de Clermont-Ferrand.

2) *Le triticale*

On a traité, avec le produit de l'exemple en 1984, 30 $F_3$ provenant de Clermont-Ferrand ainsi que 5 variétés, suivant le protocole suivant:

$F_3$: 1 dose au stade $t_1$ (= $d_2$) à la dose $d_3$, 2 stades ($t_1$ et $t_2$)

variétés: toutes traitées à $d_3$ $t_2$.

De plus, un essai triticale a été mis en place avec des $F_2$ provenant de Clermont-Ferrand. Une dose ($d_2$) a été testée à 3 stades différents ($t_3$, $t_4$, $t_4$, traitements les 4, 10, 15 mai).

Des prélèvements ont été faits pour l'orge et le triticale à chaque traitement pour détermination du stade. Quelques battages ont été faits sur triticale et montrent la présence de grains plats.

On a obtenu aussi 20 plantes de triticale à partir de culture *in vitro* d'embryons immatures.

Exemple 12

Application au maïs

Après traitement comme précédemment, on a pu observer que certains épis traités présentaient des grains à phénotype différent des grains normalement développés; ces grains anormaux étaient plus petits, parfois avec une dépression, parfois fripés. L'albumen de ces grains étaient moins abondant (parfois absent) ou qualitativement différent de l'albumen des grains normaux provenant des mêmes épis ou des témoins. Après dissection, on constatait que les embryons de ces grains étaient plus petits mais on n'a jamais observé de phénotype anormal chez ces embryons comme chez le blé.

Le cultures d'embryons ont été menées sur un milieu à 1% de saccharose, 1% d'agar et 10 mg/l de Thiamine, et les embryons ont été prélevés plus de 20 jours après la fécondation.

On a mis en culture environ 200 embryons, 103 ont été régénérés (soit environ 52%) en plantules; après sortie de tube en pots de vermiculite ces plantes ont été gardées au laboratoire dans la chambre de culture puis transportées au phytotron. 68 plantes ont survécu (soit environ 66%).

On a effectué des comptages sur les pointes de racines prélevées sur les plantules à la sortie de tube; la plupart des plantes comptées se sont révélées diploïdes. Dans quelques cas, des niveaux de ploïdie inférieurs à 2n (= 20 chromosomes)ont été observés (ex: 14, 15, 18).

ECHELLE DE FEEKES MODIFIE PAR LARGE

Définition et repère des stades de croissance des céréales d'après les caractères visibles extérieurement à l'oeil nu

| | Stade | Description | |
|---|---|---|---|
| Période des feuilles | 1 | une feuille | Tallage |
| | 2 | début du tallage | |
| | 3 | formation de talles, port rampant du blé | |
| | 4 | commencement du redressement | |
| | 5 | La pseudo-tige formée par les gaines foliaires est nettement redressée | |
| Période des tiges | 6 | premier noeud de la tige principale formé | Extension de la tige (montaison) |
| | 7 | deuxième noeud formé | |
| | 8 | dernière feuille visible mais encore enroulée, l'épi commençant à gonfler | |
| | 9 | ligule de la dernière feuille juste visible | |
| | 10 | gaine de la dernière feuille complètement sortie, épi gonflé mais non visible | |
| Période des épis | 10—1 | premiers épis juste visibles (barbes commençant à apparaître dans l'orge; épi sortant d'une fente de la gaine dans le blé et l'avoine) | Epiaison |
| | 10—2 | quart de l'épiaison achevé | |
| | 10—3 | moitié de l'épiaison achievée | |
| | 10—4 | trois quarts de l'épiaison achevés | |
| | 10—5 | tous les épis hors de la gaine | |
| | 10—5—1 | commencement de la floraison (blé) | Floraison |
| | 10—5—2 | floraison complète de l'extrémité de l'épi | |
| | 10—5—3 | floraison à la base de l'épi | |
| | 10—5—4 | amande aqueuse | Maturation |
| | 11—1 | grain laiteux | |
| | 11—2 | grain farineux, mou mais sec | |
| | 11—3 | amande dure (difficile à diviser par l'ongle) | |
| | 11—4 | bon à couper, paille morte | |

**Revendications**

1. Procédé d'obtention d'haploîdes doublés de céréales dans lequel:

a) on traite les plantes par un agent chimique d'haplodiploïdisation entre le stade de croissance de 5 à 9 selon l'échelle de Feekes modifiée par Large;

b) après pollinisation par un pollen fertile, provenant de plantes non traitées situées dans l'environnement des plantes traitées, on trie les caryopses anormaux parmi tous les caryopses obtenus;

c) on régénère les plantes à partir des embryons des caryopses anormaux, et

d) on réalise l'autofécondation de chacune des plantes régénérées pour obtenir les semences correspondant, dans certains cas, à des plantes homozygotes dans la génération suivante.

2. Procédé selon la revendication 1, caractérisé en ce que la céréale traitée est choisie parmi le blé dur, le blé commun, l'orge, le tritical, l'avoine, le sarrasin, le maîs, le riz, le sorgho et le seigle.

3. Procédé selon l'une des revendications 1 et 2, caractérisé en ce que l'étape a) est effectuée au stade de croissance 6, 7 ou 8.

4. Procédé selon l'une des revendications 1 à 3, caractérisé en ce que l'agent chimique d'haplodiploîdisation est choisi parmi les agents chimiques d'hybridation (A.C.H.), les gamétocides ou les substances de croissance.

5. Procédé selon la revendication 4, caractérisé en ce que l'agent chimique d'haplodiploïdisation est choisi parmi l'acide 1 - (4 - chlorphényle) - 1,4 - dihydro - 4 - oxo - 6 - méthylpyridazine - 3 - carboxylique et ses sels, l'acide 2-chloroéthylphosphonique et ses sels et le chlormequat et ses sels.

6. Procédé selon la revendication 4, caractérisé en ce que l'agent chimique d'hybridation utilisé est l'acide 1-(4-chlorphényle)-4-oxo-6-méthylpyridazinone-3-carboxylique et ses sels, en particulier le sel de potassium.

7. Procédé selon la revendication 4, caractérisé en ce que les plantes sont traitées à des doses d'A.C.H. de 0,04 à 10 kg/ha.

8. Procédé selon l'une des revendications 1 à 7, caractérisé en ce que les caryopses anormaux prélevés présentent un albumen réduit et possédent un embryon, dont le point de départ est une cellule haploïde.

9. Procédé selon l'une des revendications 1 à 8, caractérisé en ce que les plantes sont régénérées par culture *in vitro* de l'embryon prélevé à partir du caryopse.

10. Procédé selon l'une des revendications 1 à 8, caractérisé en ce que les plantes sont régénérées directement par germination et mise en culture de caryopses anormaux.

**Patentansprüche**

1. Verfahren zur Herstellung von Doppelhaploiden von Getreide, bei dem man:

a) die Pflanzen mit einem chemischen Mittel zur Haplodiploidbildung zwischen den Wachstumsstadien 5 bis 9 nach der Skala von Feekes, modifiziert durch Large, behandelt;

b) nach der Bestäubung mit einem fruchtbaren Pollen, der von nichtbehandelten Pflanzen aus der Umgebung der behandelten Pflanzen stammt, die anormalen Karyopsen aus allen erhaltenen Karyopsen aussortiert;

c) die Pflanzen ausgehend von Embryonen anormaler Karyopsen regeniert und

d) eine Selbstbefruchtung jeder regenerierten Pflanze durchführt, um Samen, die in bestimmten Fällen homozygoten Pflanzen in der folgenden Generation entsprechen, zu erhalten.

2. Verfahren nach Anspruch 1, dadurch gekennzeichnet, daß das behandelte Getreide ausgewählt wird aus Hartweizen, gemeinem Weizen, Gerste, Triticale, Hafer, Buchweizen, Mais, Reis, Sorghum und Roggen.

3. Verfahren nach einem der Ansprüche 1 und 2, dadurch gekennzeichnet, daß die Stufe a) im Wachstumsstadium 6,7 oder 8 durchgeführt wird.

4. Verfahren nach einem der Ansprüche 1 bis 3, dadurch gekennzeichnet, daß das chemische Mittel zur Haplodiploidbildung ausgewählt wird aus den chemischen Hybridisierungsmittel (A.C.H.), den Gametociden oder den Wachstumssubstanzen.

5. Verfahren nach Anspruch 4, dadurch gekennzeichnet, daß das chemische Mittel zur Haplodiploidbildung ausgewählt wird aus 1 - (4 - Chlorphenyl) - 1,4 - dihydro - 4 - oxo - 6 - methylpyridazin - 3 - carbonsäure und ihren Salzen, 2-Chorethylphosphonsäure und ihren Salzen, und Chlormequat und seinen Salzen.

6. Verfahren nach Anspruch 4, dadurch gekennzeichnet, daß das zur Hybridisierung verwendete chemische Mittel 1 - (4 - Chlorphenyl) - 4 - oxo - 6 - methylpyridazinon - 3 - carbonsäure und ihre Salze, insbesondere das Kaliumsalz, ist.

7. Verfahren nach Anspruch 4, dadurch gekennzeichnet, daß die Pflanzen mit A.C.H.-Mengen von 0,04 bis 10 kg/ha behandelt werden.

8. Verfahren nach einem der Ansprüche 1 bis 7, dadurch gekennzeichnet, daß die entnommenen anormalen Karyopsen ein reduziertes Albumen aufweisen und ein Embryon besitzen, dessen Ausgangspunkt eine Haploidzelle ist.

9. Verfahren nach einem der Ansprüche 1 bis 8, dadurch gekennzeichnet, daß die Pflanzen durch *in vitro* Kultur des aus der Karyopse entnommenen Embryons regeneriert werden.

10. Verfahren nach einem der Ansprüche 1 bis 8, dadurch gekennzeichnet, daß die Pflanzen direkt durch Keimen und Kultur de anormalen Karyopsen regeniert werden.

## Claims

1. A method of obtaining doubled haploids of cereals in which:

(a) the plants are treated with a chemical haplodiplodisation agent between growth stages 5 and 9, in accordance with the Feekes scale modified by Large;

(b) after pollinisation by a fertile pollen obtained from untreated plants situated in the environment of the treated plants, the abnormal caryopses are selected from all the caryopses obtained;

(c) the plants are regenerated from embryos of abnormal caryopses and

(d) each regenerated plant is self-fertilized to obtain seeds which in certain cases correspond to homozygous plants in the next generation.

2. A method according to claim 1, characterised in that the treated cereal is chosen from among hard wheat, ordinary wheat, barley, triticale, oats, buckwheat, maize, rice, sorghum and rye.

3. A method according to claim 1 or 2, characterised in that step (a) is carried out at the growth stage 6, 7 or 8.

4. A method according to any of claims 1 to 3, characterised in that the chemical haplodiploidisation agent is chosen from among chemical hybridisation agents (C.H.A.), gametocides or growth substances.

5. A method according to claim 4, characterised in that the chemical haplodiplodisation agent is chosen from among 1-(4-chlorophenyl)-1,4-dihydro-4-oxo-6-methyl pyridazine-3-carboxylic acid and salts thereof, 2-chloroethylphosphonic acid and salts thereof and chloromequate and salts thereof.

6. A method according to claim 4, characterized in that the chemical hybridisation agent used is 1-(4-chlorophenyl)-4-oxo-6-methylpyridazinone-3-carboxylic acid and salts thereof, more particularly the potassium salt.

7. A method according to claim 4, characterised in that the plants are treated with doses of from 0.04 to 10 kg of C.H.A. per ha.

8. A method according to any of claims 1 to 7, characterised in that the selected abnormal caryopses have reduced albumin and an embryo, the starting point of which is a haploid cell.

9. A method according to any of claims 1 to 8, characterised in that the plants are regenerated by cultivation *in vitro* of the embryo selected from the caryopsis.

10. A method according to any of claims 1 to 8, characterised in that the plants are regenerated directly by germination and cultivation of the abnormal caryopses.